# EUROPEAN PATENT APPLICATION

(11) **EP 4 844 621 A1**
(43) Date of publication of application: **25.11.2026**
(21) Application number: 26178308.8
(22) Date of filing: 12.05.2026
(51) Int. Cl.: G06F 30/15, G06F 30/17, G06F 30/20, B29C 65/00, G06F 113/26, G06F 119/14

(54) **METHOD AND APPARATUS FOR MANUFACTURING HYBRID ADHESIVE-RIVETED CONNECTION FOR COMPOSITE COMPONENTS**

(30) Priority: 22.05.2025 CN 202510668346
(71) Applicant: CRRC QINGDAO SIFANG CO., LTD., Qingdao, Shandong 266111 (CN)
(72) Inventor: HAN, Xiaohui, Qingdao, Shandong, 266111 (CN); XU, Lei, Qingdao, Shandong, 266111 (CN); ZHANG, Zhiyi, Qingdao, Shandong, 266111 (CN); WANG, Peng, Qingdao, Shandong, 266111 (CN); FANG, Xifeng, Qingdao, Shandong, 266111 (CN); LI, Gangqing, Qingdao, Shandong, 266111 (CN); CHEN, Yanrong, Qingdao, Shandong, 266111 (CN)
(74) Representative: Prinz & Partner mbB

(57) **Abstract**

A method and an apparatus for manufacturing hybrid adhesive-riveted connection for composite components, an electronic device and a storage medium are provided. The method includes: determining a composite parameter respectively corresponding to each type of composite components among all types of composite components in a vehicle body of a to-be-assembled vehicle; determining riveting process information respectively corresponding to each riveted structure of the riveted structures; determining a riveting procedure process parameter respectively corresponding to each riveted structure of the riveted structures; performing vehicle quality verification on the vehicle based on the composite parameter, the riveting process information and the riveting procedure process parameter; and manufacturing, in response to the vehicle quality verification being passed, the hybrid adhesive-riveted connection for composite components based on the composite parameter, the riveting process information and the riveting procedure process parameter.

## Description

### FIELD

The present disclosure relates to the technical field of riveting, and in particular to a method and an apparatus for designing a hybrid adhesive-riveted manufacturing process for composite components, an electronic device, and a computer-readable storage medium.

### BACKGROUND

With the continuous development of rail vehicles towards higher speeds and heavier loads, enhancing the energy efficiency of the rail vehicles and achieving the lightweight of trains become particularly crucial. The design and manufacturing of a lightweight train require advanced material technologies to reduce the weight of the train while ensuring its structural strength and safety. Composite materials are conducive to the development of lightweight trains due to their exceptional specific strength and specific modulus, as well as good corrosion resistance and fatigue resistance.

However, composite components exhibit characteristics such as assembly difficulties, susceptibility to damage, and low stiffness. Traditional riveting cannot satisfy manufacturing requirements. Consequently, hybrid adhesive-riveted connection serves as a primary connection manner for composite components. The hybrid adhesive-riveted connection for composite components is affected by various factors, such as inherent properties of composite materials, structural design, assembly tolerances, stress distribution, adhesive-bonding interface, and riveting state. Therefore, the reliability of connection performance of the hybrid adhesive-riveted connection during long-term service is challenged. Currently, a manufacturing methodology for hybrid adhesive-riveted connection and a complete process design method are lacking, and the performance and quality of adhesive-riveted connection cannot be ensured.

In view of this, a problem to be solved by those skilled in the art is how to provide a method and an apparatus for designing a hybrid adhesive-riveted manufacturing process for composite components, an electronic device, and a computer-readable storage medium, to ensure the performance and quality of adhesive-riveted connection.

### SUMMARY

The objective of embodiments of the present disclosure is to provide a method and an apparatus for designing a hybrid adhesive-riveted manufacturing process for composite components, an electronic device, and a computer-readable storage medium. The present disclosure provides a complete process design method that is conducive to ensuring the performance and quality of adhesive-riveted connection.

To address the above technical problem, following technical solutions are provided according to embodiments of the present disclosure.

In an aspect, the present disclosure provides a method for designing a hybrid adhesive-riveted manufacturing process for composite components, including:
determining composite information respectively corresponding to each type of composite components among all types of composite components in a vehicle body;
determining riveting process design information respectively corresponding to each riveted structure of riveted structures, where the riveting process design information includes at least two of riveting design information, procedure qualification information and procedure specification information;
determining riveting procedure process design information respectively corresponding to each riveted structure of the riveted structures;
performing vehicle quality verification on a vehicle assembled from the all types of composite components and the riveted structures based on the composite information, the riveting process design information and the riveting procedure process design information, and determining, in response to the vehicle quality verification being passed, the composite information respectively corresponding to each type of composite components among the all types of composite components, and the riveting process design information and the riveting procedure process design information respectively corresponding to each riveted structure of the riveted structures.

In an embodiment, the composite information includes at least two of a composite type, a composite molding manner and a hybrid connection manner,
where the determining composite information respectively corresponding to each type of composite components among all types of composite components in a vehicle body includes:
in response to a main component of the vehicle body being a composite vehicle body for a low-speed urban rail vehicle platform, determining that a composite type of the main component of the vehicle body is a thin-walled integral load-bearing riveted-structure composite,
   in response to the main component being an underframe, determining that a composite molding manner of the underframe is pultrusion molding, and determining that a hybrid connection manner of the underframe is adhesive-riveted connection;
   in response to the main component being a side wall, determining that a composite molding manner of a upper side sill of the side wall is pultrusion molding, determining that the composite molding manner of a side wall unit of the side wall is lay-up molding, and determining that a hybrid connection manner of the side wall is adhesive-riveted connection;
   in response to the main component being a roof, determining that a composite molding manner of the roof is lay-up molding, and determining that a hybrid connection manner of the roof is adhesive-riveted connection; and
   in response to the main component being an end wall, determining that a composite molding manner of the end wall is integrated lay-up molding, and determining that a hybrid connection manner of the end wall includes adhesive-riveted connection;
in response to the main component of the vehicle body being a composite vehicle body for a high-speed train vehicle platform, determining that the composite type of the main component of the vehicle body is the thin-walled integral load-bearing riveted-structure composite,
   in response to the main component being the underframe, determining that the composite molding manner of the underframe is pultrusion molding, and determining that the hybrid connection manner of the underframe is adhesive-riveted connection;
   in response to the main component being the side wall, determining that a composite molding manner of the side wall is pultrusion molding, and determining that the hybrid connection manner of the side wall is adhesive bonding;
   in response to the main component being the roof, determining that the composite molding manner of the roof is pultrusion molding, and determining that the hybrid connection manner of the roof is adhesive bonding;
   in response to the main component being the end wall, determining that the composite molding manner of the end wall is integrated lay-up molding, and determining that the hybrid connection manner of the end wall includes adhesive-riveted connection; and
in response to the main component of the vehicle body being a composite-material bogie component of a train, determining that the composite type of the main component of the vehicle body is a thick-walled integral load-bearing riveted-joint composite, determining that the composite molding manner of the main component of the vehicle body is interwoven winding molding, and determining that the hybrid connection manner of the main component of the vehicle body is adhesive-riveted connection.

In an embodiment, the riveting design information includes riveted-structure design information and riveted-joint design information, and
the determining riveting process design information respectively corresponding to each riveted structure of riveted structures includes:
designing the riveted structures based on a product design requirement to obtain the riveted-structure design information that satisfies the product design requirement;
designing a riveted joint based on the riveted-structure design information to obtain the riveted-joint design information;
determining a key quality characteristic based on the riveted-joint design information, and performing riveting process evaluation on the key quality characteristic to obtain the procedure qualification information; and
determining the procedure specification information based on the riveted-joint design information and the procedure qualification information.

In an embodiment, the riveting procedure process design information includes assembly accuracy information, interface adjustment process, riveting hole preparation process, and blind riveting process.

In an embodiment, the determining riveting procedure process design information respectively corresponding to each riveted structure of the riveted structures includes:
determining an assembly deviation threshold corresponding to each type of assembly component, and determining assembly deviation thresholds respectively corresponding to each type of assembly components among all types of assembly components as the assembly accuracy information;
determining that the interface adjustment process for the riveted structure includes performing, based on an assembly requirement of the riveted structure, assembly gap compensation on a connection interface of the riveted structure;
determining that the riveting hole preparation process for the riveted structure includes adding a glass fiber layer on an inner surface of a profile, and performing combined riveting hole preparation in the profile and the glass fiber layer; and
determining that the blind riveting process for the riveted structure includes determining a riveting sequence of a multi-faceted blind riveted structure, performing, based on a load displacement curve of blind riveting, quality verification on the riveting procedure after performing riveting procedure based on the riveting sequence, and removing a locked spindle blind rivet.

In an embodiment, the method further includes:
assembling and manufacturing all types of composite components in the vehicle body through a preset assembly device based on the composite information, the riveting process design information, and the riveting procedure process design information; and
evaluating the whole vehicle after the assembling and manufacturing.

In an embodiment, the evaluating the whole vehicle after the assembling and manufacturing includes:
performing quality defect verification, and/or service performance verification, and/or corrosion state verification on the vehicle after the assembling and manufacturing.

In another aspect, the present disclosure further provides an apparatus for designing a hybrid adhesive-riveted manufacturing process for composite components, including:
a first determination module, configured to determine composite information respectively corresponding to each type of composite components among all types of composite components in a vehicle body;
a second determination module, configured to determine riveting process design information respectively corresponding to each riveted structure of riveted structures, where the riveting process design information includes at least two of riveting design information, procedure qualification information and procedure specification information;
a third determination module, configured to determine riveting procedure process design information respectively corresponding to each riveted structure of the riveted structures; and
a fourth determination module, configured to perform vehicle quality verification on a vehicle assembled from the all types of composite components and the riveted structures based on the composite information, the riveting process design information and the riveting procedure process design information, and determine, in response to the vehicle quality verification being passed, the composite information respectively corresponding to each type of composite components among the all types of composite components, and the riveting process design information and the riveting procedure process design information respectively corresponding to each riveted structure of the riveted structures.

In another aspect, the present disclosure further provides an electronic device, including:
a memory, configured to store a computer program; and
a processor, configured to execute the computer program to perform the method as described above.

In another aspect, the present disclosure further provides a computer-readable storage medium, where the computer-readable storage medium stores a computer program, and the computer program, when executed by a processor, causes the processor to perform the method as described above.

It can be seen from the above technical solutions that advantages of the embodiments of the present disclosure are as follows.

A method for designing a hybrid adhesive-riveted manufacturing process for composite components is provided according to an embodiment of the present disclosure. The method includes: determining composite information respectively corresponding to each type of composite components among all types of composite components in a vehicle body; determining riveting process design information respectively corresponding to each riveted structure of riveted structures, where the riveting process design information includes at least two of riveting design information, procedure qualification information and procedure specification information; determining riveting procedure process design information respectively corresponding to each riveted structure of the riveted structures; performing vehicle quality verification on a vehicle assembled from the all types of composite components and the riveted structures based on the composite information, the riveting process design information and the riveting procedure process design information, and determining, in response to the vehicle quality verification being passed, the composite information respectively corresponding to each type of composite components among the all types of composite components, and the riveting process design information and the riveting procedure process design information respectively corresponding to each riveted structure of the riveted structures.

It can be seen that in the present disclosure, composite information respectively corresponding to each type of composite components among all types of composite components in a vehicle body is determined, and riveting process design information respectively corresponding to each riveted structure of riveted structures is determined, where the riveting process design information includes at least two of riveting design information, procedure qualification information and procedure specification information. Further, riveting procedure process design information respectively corresponding to each riveted structure of the riveted structures is determined, and a vehicle is assembled from the all types of composite components and the riveted structures based on the composite information, the riveting process design information and the riveting procedure process design information. Vehicle quality verification is further performed on the vehicle after assembly. In response to the vehicle quality verification being passed, the composite information respectively corresponding to each type of composite components among the all types of composite components, and the riveting process design information and the riveting procedure process design information respectively corresponding to each riveted structure of the riveted structures are determined. In this way, process design information at every stage is determined, facilitating the manufacture of qualified vehicles based on the determined information. A complete process design method is provided according to the present disclosure, which is conducive to ensuring the performance and quality of adhesive-riveted connection.

In addition, an apparatus, an electronic device, and a computer-readable storage medium for implementing the method for designing a hybrid adhesive-riveted manufacturing process for composite components, are provided according to the present disclosure, to further make the provided method practical. The apparatus, the electronic device, and the computer-readable storage medium exhibit the same advantages as the method.

### BRIEF DESCRIPTION OF THE DRAWINGS

To clearly describe the technical solutions in the embodiments of the present disclosure, drawings to be used in the related art or the embodiments are briefly introduced hereinafter. The drawings described below show merely some embodiments of the present disclosure, and those skilled in the art can obtain other drawings based on the provided drawings without any creative effort.
FIG. 1 is a flowchart of a method for designing a hybrid adhesive-riveted manufacturing process for composite components according to an embodiment of the present disclosure;
FIG. 2 is an overall framework diagram of a method for designing a hybrid adhesive-riveted manufacturing process for composite components according to an embodiment of the present disclosure;
FIG. 3 is a first schematic diagram of a method for using composites in a riveted structure of a rail vehicle according to an embodiment of the present disclosure;
FIG. 4 is a second schematic diagram of a method for using composites in a riveted structure of a rail vehicle according to another embodiment of the present disclosure;
FIG. 5 is a third schematic diagram of a method for using composites in a riveted structure of a rail vehicle according to another embodiment of the present disclosure;
FIG. 6 is a schematic diagram of a method and a system for designing a digital process according to an embodiment of the present disclosure;
FIG. 7 is a schematic diagram of a riveting process procedure according to an embodiment of the present disclosure;
FIG. 8 is a structural diagram of an L-shaped combined drill jig according to an embodiment of the present disclosure;
FIG. 9 is a schematic diagram of a riveting manufacturing assembling device according to an embodiment of the present disclosure;
FIG. 10 is a schematic diagram of constructing an evaluation system according to an embodiment of the present disclosure; and
FIG. 11 is a schematic structural diagram of an apparatus for designing a hybrid adhesive-riveted manufacturing process for composite components according to an embodiment of the present disclosure.

### DETAILED DESCRIPTION

A method and an apparatus for designing a hybrid adhesive-riveted manufacturing process for composite components, an electronic device, and a computer-readable storage medium are provided according to embodiments of the present disclosure. The present disclosure provides a complete process design method that is conducive to ensuring the performance and quality of adhesive-riveted connection.

To make the objective, technical solutions, and advantages of the embodiments of the present disclosure clear, the technical solutions in the embodiments of the present disclosure are to be clearly and completely described below in conjunction with the drawings in the embodiments of the present disclosure. The described embodiments are only some embodiments of the present disclosure, rather than all the embodiments. All other embodiments obtained by those skilled in the art based on the embodiments in the present disclosure without any creative efforts fall within the protection scope of the present disclosure.

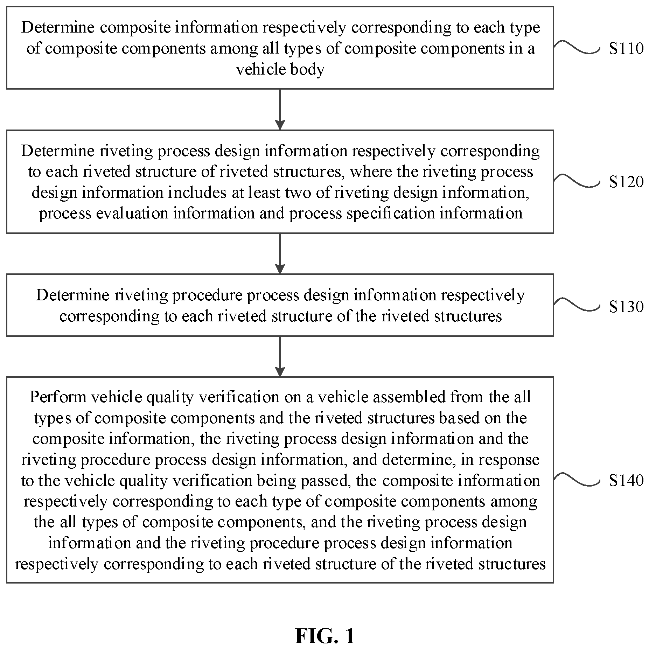

Reference is made to FIG. 1, which is a flowchart of a method for designing a hybrid adhesive-riveted manufacturing process for composite components according to an embodiment of the present disclosure. The method includes the following steps.

In step S110, composite information respectively corresponding to each type of composite components among all types of composite components in a vehicle body is determined.

In practice, composite information respectively corresponding to each type of composite components among all types of composite components in the vehicle body is determined based on anisotropy and microstructural differences of a material matrix, and structural characteristics and service environment of all types of composite components in a vehicle. The composite information includes at least two of a composite type, a composite molding manner and a hybrid connection manner.

In step S120, riveting process design information respectively corresponding to each riveted structure of riveted structures is determined, where the riveting process design information includes at least two of riveting design information, procedure qualification information and procedure specification information.

In an embodiment, for riveted structures in the vehicle body, the riveting process design information of each riveted structure of the riveted structures is determined. Specifically, the riveting design information, the procedure qualification information, and the procedure specification information are determined.

In step S130, riveting procedure process design information respectively corresponding to each riveted structure of the riveted structures is determined.

In an embodiment, for process procedure design during riveting assembly, riveting procedure process design information respectively corresponding to each riveted component of riveted components is determined. The riveting procedure process design information refers to a process and related design information adopted by the riveted component during riveting assembly.

In step S140, vehicle quality verification is performed on a vehicle assembled from the all types of composite components and the riveted structures based on the composite information, the riveting process design information and the riveting procedure process design information, and the composite information respectively corresponding to each type of composite components among the all types of composite components, and the riveting process design information and the riveting procedure process design information respectively corresponding to each riveted structure of the riveted structures are determined in response to the vehicle quality verification being passed.

After the composite information respectively corresponding to each type of composite components among the all types of composite components, and the riveting process design information and the riveting procedure process design information respectively corresponding to each riveted structure of the riveted structures are determined, each type of composite components among the all types of composite components and each riveted structure of the riveted structures are assembled based on the composite information respectively corresponding to each type of composite components among the all types of composite components, and the riveting process design information and the riveting procedure process design information respectively corresponding to each riveted structure of the riveted structures during a vehicle assembly procedure, to obtain a vehicle after assembly. Then, vehicle quality verification is performed on the assembled vehicle. In response to the vehicle quality verification being passed, it is indicated that by perform vehicle assembling based on the determined composite information, the determined riveting process design information, and the determined riveting procedure process design information, a qualified vehicle is obtained. Therefore, the determined composite information, the determined riveting process design information, and the determined riveting procedure process design information are taken as final information adopted in the vehicle assembly procedure, thereby ensuring the performance and quality of the adhesive-riveted connection in the vehicle.

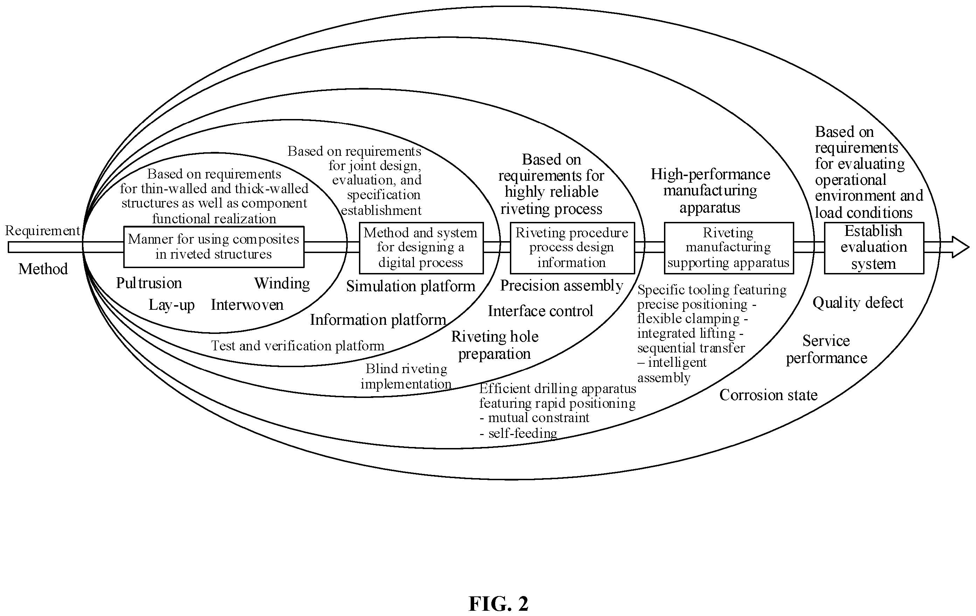

The method for designing a hybrid adhesive-riveted manufacturing process for composite components provided in the embodiment of the present disclosure includes multiple stages, namely: a manner for using composites in riveted structures (that is, determining the composite information of the composite components), a digital process design manner (which is used for determining the riveting process design information), a riveting procedure process procedure (which is used for determining the riveting procedure process design information), a riveting manufacturing supporting apparatus (which is used for vehicle assembly), and a construction manner for an evaluation system (which is used for quality evaluation of the assembled vehicle). These five manners are illustrated in FIG. 2.

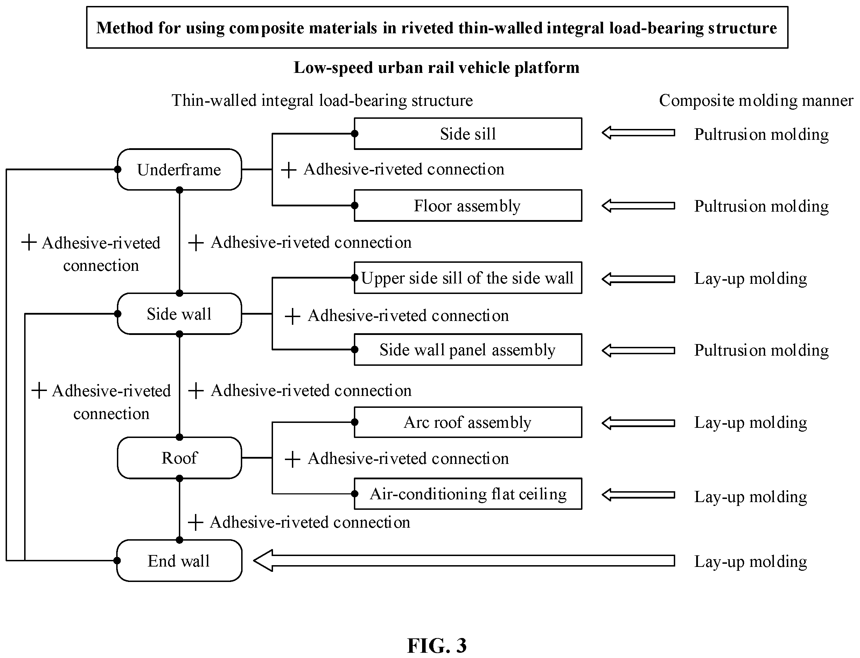

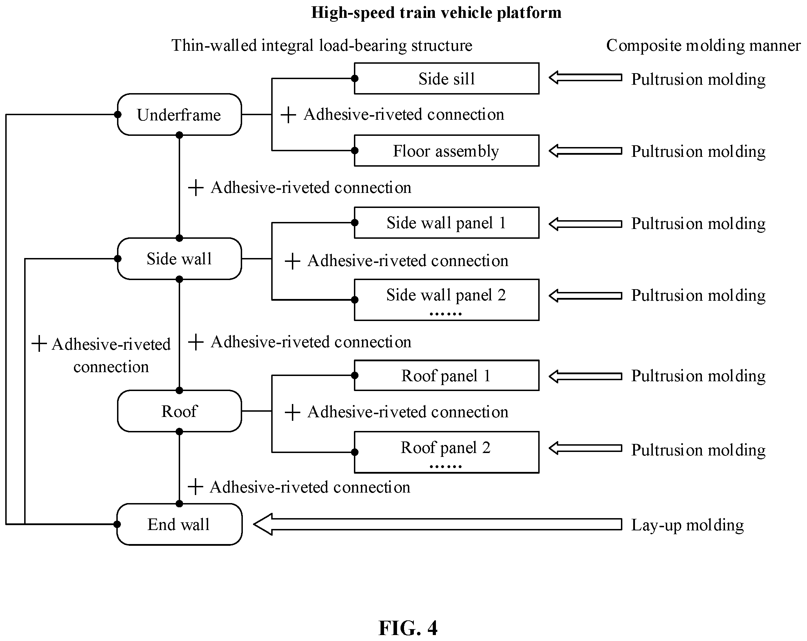

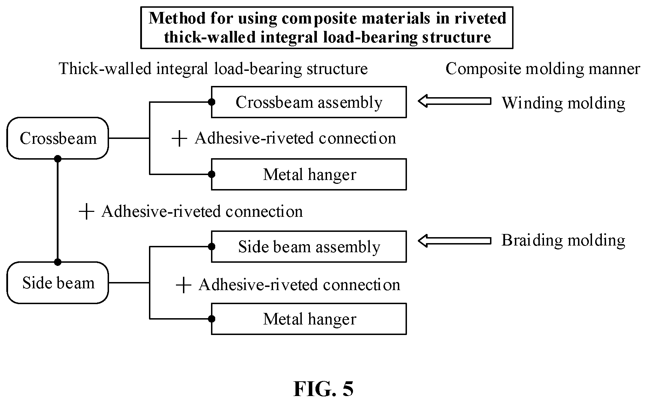

In the present disclosure, the method for using composite materials is developed based on the anisotropy and microstructural differences of the material matrix, and structural characteristics and service environment of the vehicle, to determine the composite information of all types of composite components in the vehicle. According to different scenarios and service requirements, composite molding manners employed for the composite components in the vehicle primarily include pultrusion molding, lay-up molding and interwoven winding molding. Pultrusion-molded composites are used for thin-walled, large and long profile components with uniform cross-sections. Lay-up-molded composites are used for complex-structured thin-walled plate and beam components. Weaved composite components are used for thick-walled and high-bearing structures. The applications of various components in the vehicle are illustrated in FIG. 2 to FIG. 5. Hereinafter the technical solution is further explained and introduced.

In an embodiment, the composite information includes at least two of a composite type, a composite molding manner and a hybrid connection manner.

In this embodiment, in the above step S110, the determining composite information respectively corresponding to each type of composite components among all types of composite components in a vehicle body may include:
in response to a main component of the vehicle body being a composite vehicle body for a low-speed urban rail vehicle platform, determining that a composite type of the main component of the vehicle body is a thin-walled integral load-bearing riveted-structure composite,
   in response to the main component being an underframe, determining that a composite molding manner of the underframe is pultrusion molding, and determining that a hybrid connection manner of the underframe is adhesive-riveted connection;
   in response to the main component being a side wall, determining that a composite molding manner of a upper side sill of the side wall is pultrusion molding, determining that the composite molding manner of a side wall unit of the side wall is lay-up molding, and determining that a hybrid connection manner of the side wall is adhesive-riveted connection;
   in response to the main component being a roof, determining that a composite molding manner of the roof is lay-up molding, and determining that a hybrid connection manner of the roof is adhesive-riveted connection;
   in response to the main component being an end wall, determining that a composite molding manner of the end wall is integrated lay-up molding, and determining that a hybrid connection manner of the end wall includes adhesive-riveted connection;
in response to the main component of the vehicle body being a composite vehicle body for a high-speed train vehicle platform, determining that the composite type of the main component of the vehicle body is the thin-walled integral load-bearing riveted-structure composite,
   in response to the main component being the underframe, determining that the composite molding manner of the underframe is pultrusion molding, and determining that the hybrid connection manner of the underframe is adhesive-riveted connection;
   in response to the main component being the side wall, determining the composite molding manner of the side wall as pultrusion molding, and determining that the hybrid connection manner of the side wall is adhesive bonding;
   in response to the main component being the roof, determining that the composite molding manner of the roof is pultrusion molding, and determining that the hybrid connection manner of the roof is adhesive bonding;
   in response to the main component being the end wall, determining that the composite molding manner of the end wall is integrated lay-up molding, and determining that the hybrid connection manner of the end wall includes adhesive-riveted connection; and
in response to the main component of the vehicle body being a composite-material bogie component of a train, determining that the composite type of the main component of the vehicle body is a thick-walled integral load-bearing riveted-joint composite, determining that the composite molding manner of the main component of the vehicle body is interwoven winding molding, and determining that the hybrid connection manner of the main component of the vehicle body is adhesive-riveted connection.

A thin-walled integral load-bearing structure based on the thin-walled integral load-bearing riveted-structure composite is primarily applied to a composite vehicle body of a train, which includes a low-speed urban rail vehicle platform and a high-speed train vehicle platform.

For the low-speed urban rail vehicle platform, main components of the composite vehicle body include an underframe, side walls (including a side wall one and a side wall two), a roof and an end wall. The underframe includes two types of subcomponents: side sills and floors, all of which are fabricated using pultrusion molding and are connected via adhesive-riveted connection to form an integral underframe component. The side walls include two types of subcomponents: upper side sills and side wall units. The upper side sills are formed through pultrusion molding, and the side wall units are fabricated using lay-up molding. The side walls are then connected via adhesive-riveted connection to form components being the side wall one and the side wall two. The roof includes arc roof assemblies and air-conditioning flat ceilings, all of which are fabricated using the lay-up molding process and are connected via adhesive-riveted connection to form roof subcomponents. The end wall is fabricated using integral lay-up molding. Integral structural connection formed by the main components of the vehicle body include: connection between the underframe and the side wall, connection between the underframe and the end wall, connection between the side wall and the roof, connection between the side wall and the end wall, and connection between the roof and the end wall, all of which employ hybrid adhesive-riveted connection. A metal hanger on each main component of the vehicle body is connected to the main structure of the composite vehicle body using hybrid adhesive-riveted connection.

For the high-speed train vehicle platform, main components of the composite vehicle body also include an underframe, side walls (also including a side wall one and a side wall two), a roof and an end wall. The respective composite molding manners and respective composite connection manners for all subcomponents in the composite vehicle body are described below. The underframe includes two types of subcomponents: side sills and floors, all of which are fabricated using pultrusion molding and are connected via adhesive-riveted connection to form an integral underframe component. The side walls are composed of multiple side wall panels, all of which are produced using pultrusion molding and are connected through adhesive bonding to form components being the side wall one and the side wall two. The roof is composed of multiple side wall panels, all of which are fabricated using the pultrusion molding process and are connected through adhesive bonding to form roof subcomponents. The end wall is also fabricated using integral lay-up molding. Integral structural connection formed by the main components of the vehicle body for the high-speed train vehicle platform is the same as this for the low-speed urban rail vehicle platform. A metal hanger on each main component of the vehicle body is connected to the main structure of the composite vehicle body using hybrid adhesive-riveted connection.

In addition, for a thick-walled integral load-bearing riveted-joint composite, it is used to fabricate a thick-walled integral load-bearing structure. The thick-walled integral load-bearing structure is primarily applied to a composite bogie component of the train, including crossbeams and side beams. The side beams include a side beam 1 and a side beam 2. All the side beams are molded by interwoven winding molding, the crossbeams and the side beams are connected via adhesive-riveted bonding, and the metal hangers are connected to the crossbeams and the side beams via adhesive-riveted bonding, to form a thick-walled integral load-bearing bogie component.

In an embodiment, the riveting design information includes riveted-structure design information and riveted-joint design information.

In this case, the determining riveting process design information respectively corresponding to each riveted structure of riveted structures includes: designing the riveted structures based on a product design requirement to obtain the riveted-structure design information that satisfies the product design requirement; and designing a riveted joint based on the riveted-structure design information to obtain the riveted-joint design information.

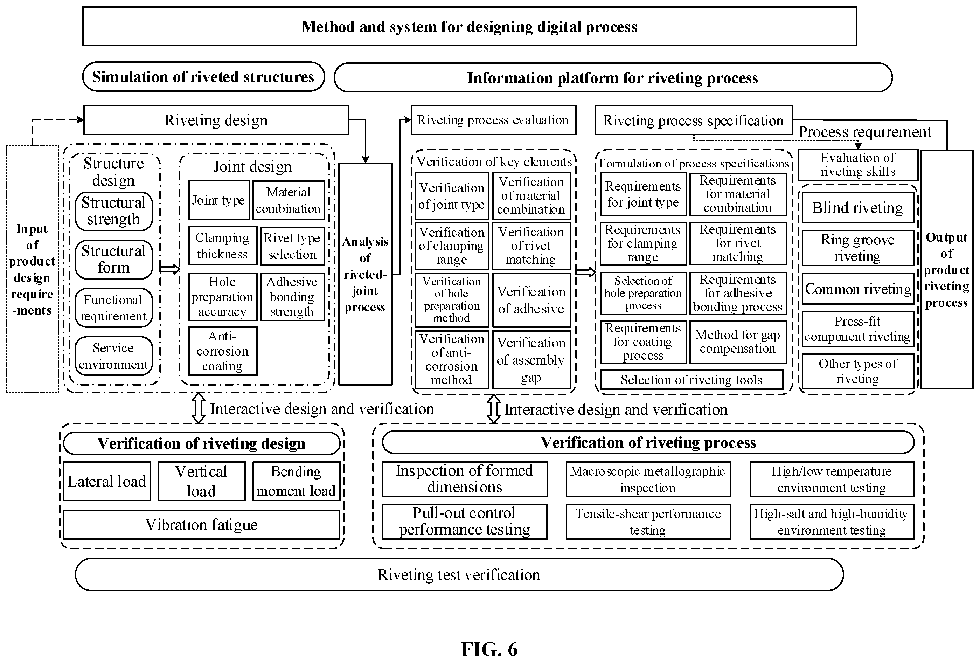

The riveting design includes two parts: riveted-structure design and riveted-joint design. In the present disclosure, the riveting design information includes the riveted-structure design information and the riveted-joint design information. The objective of this embodiment is adopting digital means to perform riveting design (including structure design and joint design), perform process evaluation verification, establish a process specification, and perform skill evaluation, thereby enabling online management of "design + process + skill." As shown in FIG. 6, in this embodiment, by analyzing the manufacturing procedure of adhesive-riveted composite connection for composite materials, a method of "simulation + information platform + experimental verification" is used, and a digital process design procedure is formed, which involves riveting design, process evaluation, specification establishment, skill evaluation, and the like.

In an embodiment, a riveted structure is designed based on product design requirements, focusing on factors such as composite structure strength, structural form, functional requirements and service environment. For example, a designer performs riveting design by adopting digital means based on relevant technical requirements and standards. The product design requirements include: dimensional requirements, strength requirements, material requirements, structural form requirements, functional requirements, and key operational requirements for the vehicle body (components including underframes, side walls, roofs, end walls, and the like) and framework (components such as side beams and crossbeams). After the riveted-structure design is finished, simulation is performed on the riveted structure based on the riveted-structure design to further verify the rationality of the structure design. In addition, after the riveted-structure design information is obtained, the riveted joint is designed based on the riveted-structure design information and a design requirement for the riveted structure, to obtain the riveted-joint design information. The riveted-joint design information includes riveted joint type, material combination, clamping thickness, rivet selection, hole preparation precision, adhesive bonding strength, anti-corrosion coating design, and the like. In addition, after the riveted-joint design is finished, static strength check including lateral loads, vertical loads, and bending moment loads may be performed for the riveted-joint design. Riveted-structure verification, such as fatigue vibration, is performed based on load conditions to ensure the rationality of the riveted-joint design and the vehicle quality. A key quality characteristic is determined based on the riveted-joint design information, and riveting process evaluation is performed on the key quality characteristic to obtain the procedure qualification information.

In practice, the riveted joint may be analyzed. A key quality characteristic is extracted based on the riveted-joint design information, and riveting process evaluation is performed based on the key quality characteristics. For every riveted point specified in the design requirements, a corresponding process method is established. A method for evaluating a riveting process is designed, and experimental verification is performed to determine the rationality of the designed method for evaluating a riveting process. A process evaluation report (i.e., the procedure qualification information) is generated based on a verification result. In practice, the experimental verification includes joint type verification, material combination verification (such as steel, aluminum, copper, and composite materials), clamping range verification, rivet matching verification, hole preparation method verification (such as match drilling or machine drilling), adhesive selection verification, anti-corrosion method verification, assembly gap verification, and the like. The joint types include blind riveting (such as bulb-type blind rivets), ring groove riveting (such as break-off type and wide-range type ring groove rivets), common riveting (such as riveting through solid rivets and hollow rivets), and press-fit component riveting (such as rivet nut riveting). In addition, riveted joint test specimens used to simulate operating conditions are fabricated to perform tests such as molding dimension inspection, macroscopic metallographic examination, tensile-shear performance testing, pull-off performance testing, high-temperature and low-temperature environmental testing, and high-salt high-humidity environmental testing, to verify the feasibility of the riveting process. The procedure specification information is determined based on the riveted-joint design information and the procedure qualification information.

The riveting process specification is a critical technical document for guiding riveting operations. In practice, a reasonable riveting process specification is established based on a riveting process evaluation result. A template for the riveting process specification is established in advance. The template primarily includes joint type requirements, material combination requirements, clamping range requirements, rivet matching requirements, hole preparation process requirements, coating process requirements, gap compensation requirements, and riveting tool selection, and the like. Information about joint type requirements, material combination requirements, clamping range requirements, rivet matching requirements, hole preparation process requirements, coating process requirements, gap compensation requirements, and riveting tool selection is extracted based on the riveted-joint design information and the riveting procedure qualification information, and then is filled into the template for the riveting process specification. In addition, the identification number of the riveting process evaluation report is filled into a corresponding section of the riveting process specification. The riveting process specification extracts 33 core elements across six key aspects: base material, adhesive, hole preparation, rivet collar, riveted joint, and riveting process. Base material element information may include material type information, thickness information, coating information, pre-drilled hole diameter information, and surface treatment information. Adhesive element information may include information about adhesive model, adhesive application position, operating time, curing time, and the like. Hole preparation element information may include information about hole diameter, hole preparation manner, hole type, countersink diameter, and countersinking manner. Riveted joint element information involves two aspects: joint design and joint preparation, where the joint design includes joint code and joint schematic diagram information (that is, 2 elements), and the joint preparation may include schematic diagram information and gap (that is, 2 elements). Riveting process element information involves three aspects: riveting method, rivet gun information, and riveting position, where the riveting method includes installation method, riveting type, riveting classification, and riveting manner (that is, 4 elements), the rivet gun information includes rivet gun name, rivet gun model, nose assembly model, and power manner (that is, 4 elements), and the riveting position includes position name (that is, 1 element). In the present disclosure, by extracting specification information and identifying aggregation relationships among elements, a riveting process specification element aggregation document is created. Previously dispersed riveting process specification elements are aggregated into a single document, which facilitates practical guidance and application. Based on the information extraction process and aggregation relationships among elements in the riveting process specification, a digital process design methodology is established. By using the riveting process specification, the elements of the riveting process specification are aggregated, and structural riveting process design and data aggregation are performed with full-element riveting process parameters.

In addition, considering that the manufacturing of riveted products requires a riveting operator to carry out construction in accordance with the established riveting process, riveting skills constitute a crucial aspect of ensuring the quality of riveted products. In this embodiment, the riveting skills of an operator may be verified through riveting skill evaluation. In an embodiment, skill evaluation is performed on riveting processes corresponding to various riveting types involved in the riveting process, ensuring that each operational skill is adapted to the riveting process specification. An updated riveting process specification is formulated to guide the production of riveted products. The various riveting types include blind riveting (such as bulb-type blind rivets), ring groove riveting (such as break-off type and wide-range type ring groove rivets), common riveting (such as riveting through solid rivets and hollow rivets), and press-fit component riveting (such as rivet nut riveting).

In an embodiment, the riveting procedure process design information includes assembly accuracy information, interface adjustment process, riveting hole preparation process, and blind riveting process.

In this embodiment, a process method for the riveting assembly procedure is determined for riveted components. The assembly procedure includes vehicle body assembly (including the assembly of underframe components, side wall components, roof components, end wall components, and the interconnection among them) and frame assembly (including the assembly of crossbeam components, side beam components, and the interconnection between crossbeams and side beams). The establishment of the process method is supported by technological requirements for riveted joints.

In this embodiment, when determining the riveting procedure process design information, a series of riveting process methods are developed primarily centered around the precise assembly of large composite components (i.e., assembly accuracy information), interface control, riveting hole preparation, and blind riveting implementation.

In an embodiment, the determining riveting procedure process design information respectively corresponding to each riveted structure of the riveted structures includes: determining an assembly deviation threshold corresponding to each type of assembly component, and determining assembly deviation thresholds respectively corresponding to each type of assembly components among all types of assembly components as the assembly accuracy information.

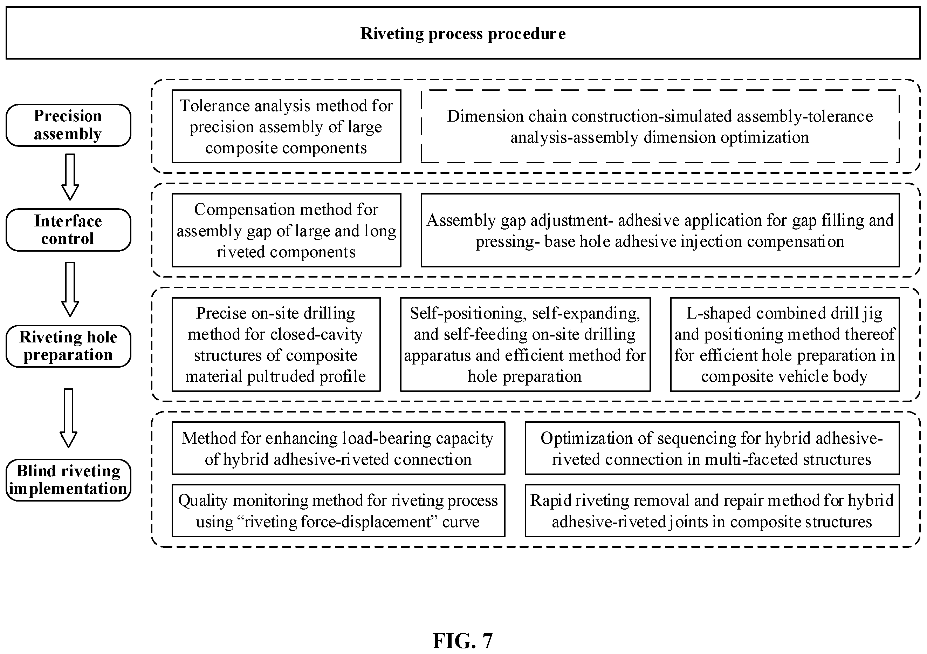

As shown in FIG. 7, in an embodiment of the present disclosure, the assembly accuracy information is determined in advance. In an embodiment, for the assembly procedure of large composite vehicle body structures, assembly deviation thresholds (such as length tolerances for an underframe component and overall height or width tolerances of the vehicle) corresponding to all types of manufacturing deviations for each assembled component are determined in advance based on manufacturing deviations such as flatness and dimensional accuracy of each assembled component, as well as tooling positioning errors. In addition, traditional assembly procedures can also be optimized based on this embodiment, to develop a tolerance analysis method for the precise assembly of large composite components, which can perform multi-stage assembly process simulation for different manufacturing deviations (such as straightness, flatness, and dimensional accuracy) of large composite components, and predict assembly errors in large composite structures under different manufacturing deviations.

The interface adjustment process for the riveted structure includes performing, based on an assembly requirement of the riveted structure, assembly gap compensation on a connection interface of the riveted structure.

In practice, interface control of connected surfaces is achieved by assembly gap compensation, to satisfy assembly requirements for riveted workpieces. The connected surfaces refer to interfaces between an upper plate of the riveted structure and an adjacent plate of the same riveted structure. The procedure of interface control of connected surfaces through assembly gap compensation involves three key steps: assembly gap adjustment, adhesive application for gap filling and pressing, and base hole adhesive injection compensation. After the adhesive cures, gaps at the riveting positions are fully compensated, thereby achieving gap elimination.

In an embodiment, when performing assembly gap compensation on large and long riveted components, the riveting assembly procedure and the riveted hole matching and drilling procedure may be combined. By pre-machining base holes at riveted hole positions on the upper plate, functions such as component assembly positioning, gap measurement, adhesive injection filling, and matching drilling can be achieved. A highly flowable structural adhesive is used in conjunction with rivets to fill irregular gaps. The composite connection formed by the adhesive-riveted joint enhances the overall strength of the riveted joint, addressing the limitations of mechanical riveting in terms of sealing performance. In addition, electrical potential isolation is provided for connection between dissimilar materials (such as connection between carbon fiber and steel components), and the corrosion risk is reduced. In practice, the arrangement of base holes resolves challenges related to positioning, gap measurement, gap compensation, and matching drilling positioning during assembly. The adhesive effectively addresses the difficulty of eliminating irregular gaps, thereby improving positioning accuracy during assembly, enabling precise gap measurement and compensation, facilitating match drilling positioning, and ultimately eliminating gaps.

The riveting hole preparation process for the riveted structure includes adding a glass fiber layer on an inner surface of a profile, and performing combined riveting hole preparation in the profile and the glass fiber layer.

In practice, riveting hole preparation is performed by using a precise on-site drilling method for closed-cavity structures of composite material pultruded profiles, ensuring high hole preparation accuracy in a closed cavity of composite side sill profiles. In an embodiment, a glass fiber layer is added to the internal surface of the profile, effectively creating a backing plate within a hollow cavity inside the pultruded profile. This provides internal surface support during composite drilling, and functions as a drill backing plate to minimize the occurrence of burrs and splitting at a hole exit point. In addition, a dedicated drill jig is designed to create riveted holes for "metal + carbon fiber hollow profiles" with base holes, enabling rapid positioning and clamping. By using an automatic feed drill unit (ADU) instead of manual electric drills for match drilling, controlled rotational speed and stable feed rates are ensured during the drilling procedure, thereby guaranteeing the accuracy of hole preparation.

In the present disclosure, the accuracy and efficiency of drilling for multi-hole and large-hole matching between composite floors and aluminum alloy connection can be enhanced by using a self-positioning, self-expanding, and self-feeding on-site drilling apparatus and an efficient method for hole preparation. In an embodiment, high-precision riveted hole preparation is achieved through a combination of concentric lightening holes, self-expanding drill jigs, and an ADU feed drill. This enables precise positioning for multi-position and multi-hole matching drilling across different materials, and precise drilling for large-diameter hole matching in thick plate assemblies. In an embodiment, several concentric lightening holes are set on the workpiece in advance. The expanding chuck of the automatic feed drill is moved into a blind hole, the expanding chuck expands and tightens against an inner wall of the blind hole. Then, a drill bit of the automatic feed drill is activated to drill a riveted hole at the bottom of the blind hole. By expanding and tightening the expanding chuck within the blind hole, the stability of the expanding chuck is ensured without any additional clamping device for holding the expanding chuck. This effectively reduces drilling costs and ensures the stable operation of the automatic feed drill, thereby ensuring drilling accuracy. Furthermore, during drilling, positioning of the expanding chuck can be achieved by moving the expanding chuck and the drill bit into the blind hole without any additional apparatus, thereby enhancing drilling efficiency.

In the present disclosure, by using an L-shaped combined drill jig and positioning method thereof for efficient hole preparation in a composite vehicle body, the matching drilling accuracy of transverse positions of riveted holes between carbon fiber side walls and aluminum alloy side sill profiles is ensured. In an embodiment, for preparation of riveted holes in large composite side wall components and large composite underframe side sill components of a vehicle, an L-shaped fixed drill jig set is designed and fabricated in the present disclosure. The L-shaped fixed drill jig set consists of two sets of L-shaped fixed bases and multiple sets of strip-shaped drill jigs that cooperate with the L-shaped fixed bases. Cooperation of the sets of L-shaped fixed base and the ADU device enables rapid installation and hole preparation. Based on different layouts of the base holes, strip-shaped drill jigs with corresponding specifications are matched, and the automatic feed drill is fixed. High-precision preparation of countersinks for the connection between side walls and underframe side sills is achieved through an integrated drill-and-countersink bit.

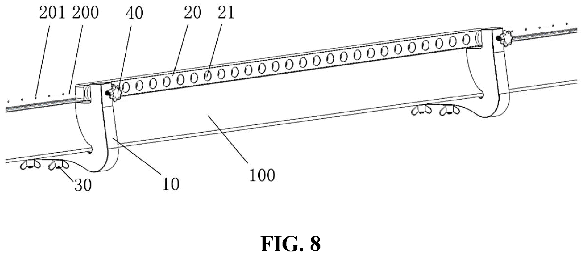

A structural diagram of the L-shaped combined drill jig for efficient hole preparation in the composite vehicle body can be referred to FIG. 8. The structure includes an L-shaped fixed base (10). The L-shaped fixed base (10) includes a horizontal mounting portion (11) and a vertical support fixing portion (12). The horizontal mounting portion (11) is used to connect to the bottom of a side sill (100) via a first fastener (30). A contour support portion (121) is provided at a side of the vertical support fixing portion (12) facing an outer surface of the side sill (100). The contour support portion (121) is used to fit against the outer surface of the side sill (100). A drill jig fixing slot (122) is provided at an upper end of the vertical support fixing portion (12) facing the side wall (200). The structure further includes a strip-shaped drill jig (20). Multiple drill bit fixing holes (21) distributed along a length direction of the strip-shaped drill jig (20) are arranged on the strip-shaped drill jig (20). The drill jig fixing slot (122) is used to accommodate the strip-shaped drill jig (20). The L-shaped fixed base (10) is used to press the strip-shaped drill jig (20) against the side wall (200). During installation, the L-shaped fixed base is placed on the side sill, such that the horizontal mounting portion fits against the bottom of the side sill, and the vertical support portion fits against the outer surface of the side wall. Then, the first fastener is inserted through the horizontal mounting portion and the bottom of the side sill to fix the L-shaped fixed base. Next, the strip-shaped drill jig is placed in the drill jig fixing slot. Finally, the drill jig is pressed against the side wall. After the strip-shaped drill jig is fixed, drilling operations can be performed. The use of the L-shaped fixed base effectively enhances the stability of the strip-shaped drill jig, thereby improving drilling accuracy.

The blind riveting process for the riveted structure includes determining a riveting sequence of a multi-faceted blind riveted structure, performing, based on a load displacement curve of blind riveting, quality verification on the riveting procedure after performing riveting procedure based on the riveting sequence, and removing a locked spindle blind rivet.

In practice, plasma surface treatment can be performed on surfaces of rivet components and adhesive-coated surfaces of composites to enhance the mechanical strength and the bonding strength of riveting, thereby improving the load-bearing performance of the hybrid adhesive-riveted connection. In addition, the stress-strain distribution within the riveted structure is coordinated to optimize the riveting sequence for the multi-faceted blind riveted structure. In practice, when solving the problem of overall deformation during riveting of small bent components, a "local-to-global" mapping loading method for structural riveting deformation is employed based on a single-rivet riveting model. This method transfers the complex stress-strain states around the riveted holes to a multi-faceted blind riveting model, enabling the prediction of riveting deformation and residual stresses in the multi-faceted blind riveted structure. In addition, with a goal of minimizing riveting deformation, the riveting sequence is optimized, thereby determining the riveting sequence.

Then, by utilizing the load-displacement curves obtained from blind riveting between homogeneous composite materials or heterogeneous composite/metal materials, and comparing them with a tolerance band for riveting quality assessment, quality monitoring can be performed during the riveting procedure. In an embodiment, small-sample blind riveting tests are performed on homogeneous composite materials or heterogeneous composite/metal materials. During the tests, the load-displacement curves of qualified riveting process are recorded and selected. Then, data processing methods such as a least squares method and an averaging method are employed to derive a standard assessment curve. Finally, a t-statistical distribution is used to determine its tolerance band (at a 95% confidence level). The tolerance band obtained through this method is validated through numerous blind riveting tests and has good accuracy, which can enhance the accuracy and efficiency of riveting quality assessment.

In practice, rivet removal and repair may be performed on composite hybrid adhesive-riveted joints. The rivet removal procedure can be simplified into two stages: rivet decomposition and rivet expulsion, to rapidly remove a locked spindle blind rivet. In an embodiment, a six-step procedure "drilling a center hole - drilling the locked spindle - punching out a rivet shank - drilling a rivet sleeve - extracting a rivet head - punching out the rivet sleeve" included in a traditional rivet removal may be streamlined into a two-step procedure "rivet decomposition - rivet expulsion" by using a "multi-stage feed combined decomposition drill". The "multi-stage feed combined decomposition drill" includes two methods being two-stage feed and three-stage feed, and a positioning and rotational speed control apparatus for the drilling tool. The drilling of the center hole, the locked spindle and the rivet sleeve is integrated into one procedure, that is, the rivet decomposition procedure. The punching out of the rivet shank and the rivet sleeve is combined into another procedure, that is, the rivet expulsion process. The entire procedure is convenient and efficient, enabling the rapid removal of locked spindle blind rivets.

In an embodiment, the method further includes:
assembling and manufacturing all types of composite components in the vehicle body through a preset assembly device based on the composite information, the riveting process design information, and the riveting procedure process design information, which facilitates improving the assembly efficiency and the assembly precision; and
evaluating the whole vehicle after the assembling and manufacturing.

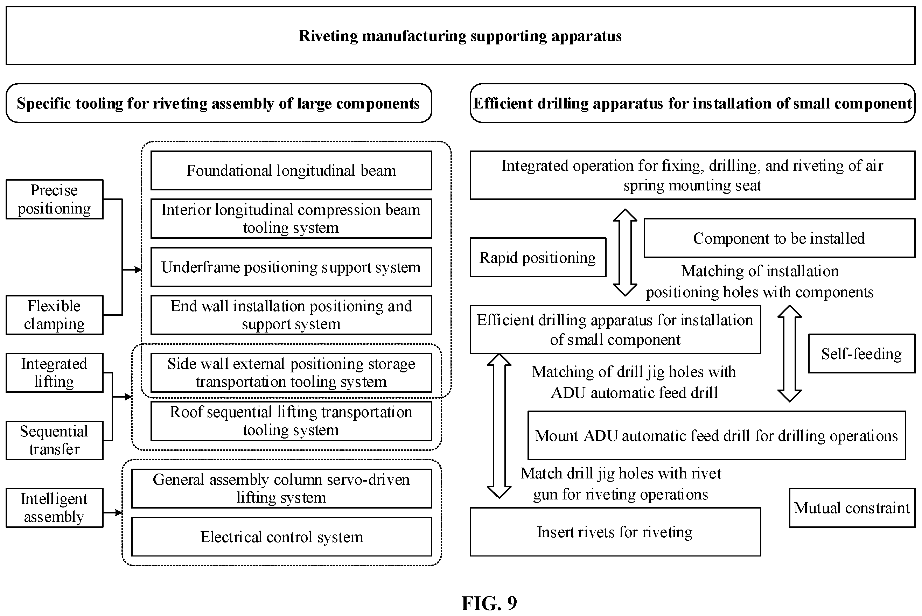

In practice, an assembly device may be designed in advance. In an embodiment, specific tooling featuring precise positioning - flexible clamping - integrated lifting - sequential transfer - intelligent assembly is developed in advance, to fabricate a series of riveting manufacturing supporting apparatuses such as efficient drilling apparatuses featuring rapid positioning - mutual constraint - self-feeding. As illustrated in FIG. 9, the riveting manufacturing supporting apparatus include specific tooling for the riveted assembly of large components and an efficient drilling apparatus for the installation of small components.

In an embodiment, the specific tooling for the riveted assembly of large components is designed to facilitate pre-assembly, disassembly, adhesive application, secondary assembly, and curing blind-riveting operations for the underframe, end walls, side walls, roof, and front end of the vehicle body. The tooling includes foundational longitudinal beams, an underframe positioning support system (including positioning support apparatuses for the bolster, and support apparatuses for the side sills, and the like), an interior longitudinal compression beam tooling system, a side wall external positioning storage transportation tooling system, an end wall installation positioning support system, a roof sequential lifting transportation tooling system, a general assembly column servo-driven lifting system, and various support rods, mobile storage racks, clamps, and electrical control systems, which form the specific tooling featuring precise positioning - flexible clamping - integrated lifting - sequential transfer - intelligent assembly together.

In practice, the pre-designed tooling device can be tailored to satisfy requirements of multiple times of assemblies, curing maintenance, and high-precision synthesis in adhesive-riveted process based on process requirements. In addition, the lifting tooling for large components adopts an integral frame structure, characterized by high strength and rigidity. The lifting tooling is configured with built-in positioning and clamping apparatuses that enable precise positioning and secure clamping of large components, ensuring product accuracy and preventing deformation during transportation. The lifting tooling is configured with high-precision interfaces that are compatible with servo-driven fetal position devices. Through interface positioning and clamping, positioning and clamping of the product is achieved. By adopting external positioning and clamping for large components instead of internal coarse adjustment positioning, interior space of the vehicle is maximized to avoid manual adjustments and measurements. By adopting a high-precision servo-driven apparatus controlled by PLC programs, high-precision synthesis of the product and automatic sequence transfer are achieved. The entire procedure is electrically controlled, with individual product coordinate correction and memory capabilities, enabling precise assembly of each product. The integral frame fixture for large components is involved throughout the entire procedure, maintaining the dimensional accuracy of the product and preventing deformation. In addition, a certain amount of pressure is applied during curing to enhance the curing performance and preserve the overall dimensional accuracy of the overall vehicle.

For example, the tooling may include a support assembly, an underframe positioning assembly, an end-wall positioning assembly, a roof positioning assembly, a side wall one positioning assembly, a side wall two positioning assembly and a controller. The underframe positioning assembly is arranged on the support assembly to provide support for the underframe. The end-wall positioning assembly is mounted on the support assembly, and is used to abut against a vertically positioned end wall, ensuring proper alignment during assembly. An output end of the roof positioning assembly is detachably connected to the roof and the underframe, and is capable of moving longitudinally, laterally, and vertically, to guide the roof and the underframe into their correct assembly positions or to lift the roof away from its original position. An output end of the side wall one positioning assembly is detachably connected to the side wall one, and is capable of moving longitudinally, laterally, and vertically to position the side wall one correctly during assembly or to move it away from its original position. An output end of the side wall second positioning assembly is detachably connected to the side wall two, and is capable of moving longitudinally, laterally, and vertically to position the side wall two correctly during assembly or to move it away from its original position. The controller is signal-connected to the roof positioning assembly, the side wall one positioning assembly, and the side wall second positioning assembly. The controller controls the resetting of the roof, the side wall one, and the side wall two during the secondary assembly of the vehicle body.

The support assembly is used to mount and support components such as the underframe positioning assembly and the end-wall positioning assembly. The underframe positioning assembly is used to support the underframe. The end-wall positioning assembly is flexibly connected to the support assembly, to abut against and restrict the position of the end wall in a Z direction. The support assembly and the end-wall positioning assembly employ a frame structure or a base design. The output end of the roof positioning assembly is capable of moving along a Y direction, an X direction, and a Z direction of the vehicle body, and can stably connect to the roof, to guide the movement of the roof along the Y direction, the X direction, and the Z direction of the vehicle body. Similarly, the output end of the side wall one positioning assembly is capable of moving along the Y direction, the X direction, and the Z direction of the vehicle body, and can stably connect to the side wall one arranged along the Z direction, to guide the movement of the side wall one along the Y direction, the X direction, and the Z direction of the vehicle body. The output end of the side wall second positioning assembly is capable of moving along the Y direction, the X direction, and the Z direction of the vehicle body, and can stably connect to the side wall two arranged along the Z direction, to guide the movement of the side wall two along the Y direction, the X direction, and the Z direction of the vehicle body.

Correspondingly, the controller is signal-connected to the roof positioning assembly, the side wall one positioning assembly, and the side wall second positioning assembly. This enables the controller to precisely control the movement of the roof, the side wall one, and the side wall two along the Y direction, the X direction, and the Z direction of the vehicle body, thereby achieving vehicle assembly.

In addition, for the efficient drilling apparatus for the installation of small components, a high-efficiency drilling apparatus featuring rapid positioning - mutual constraint - self-feeding is developed for the fixation and riveting of an air spring mounting seat to a bolster on the vehicle body. The apparatus integrates the functions of fixing the air spring mounting seat, drilling holes, and riveting into one operation. First, the air spring mounting seat is placed at a bolster belt mounting location. Then, a multifunctional drill jig is positioned over the air spring mounting seat, and the and the air spring mounting seat are fixed together onto the bolster of the underframe. Next, a self-expanding drill jig is inserted into the drill jig holes, and an ADU automatic feed drill is mounted for drilling operations. Finally, rivets are inserted for riveting. This method enables an integrated operation of component fixation, drilling, and riveting, thereby streamlining the operation procedure and enhancing installation efficiency.

In an embodiment, the evaluating the whole vehicle after the assembling and manufacturing may include:
performing quality defect verification, and/or service performance verification, and/or corrosion state verification on the vehicle after the assembling and manufacturing.

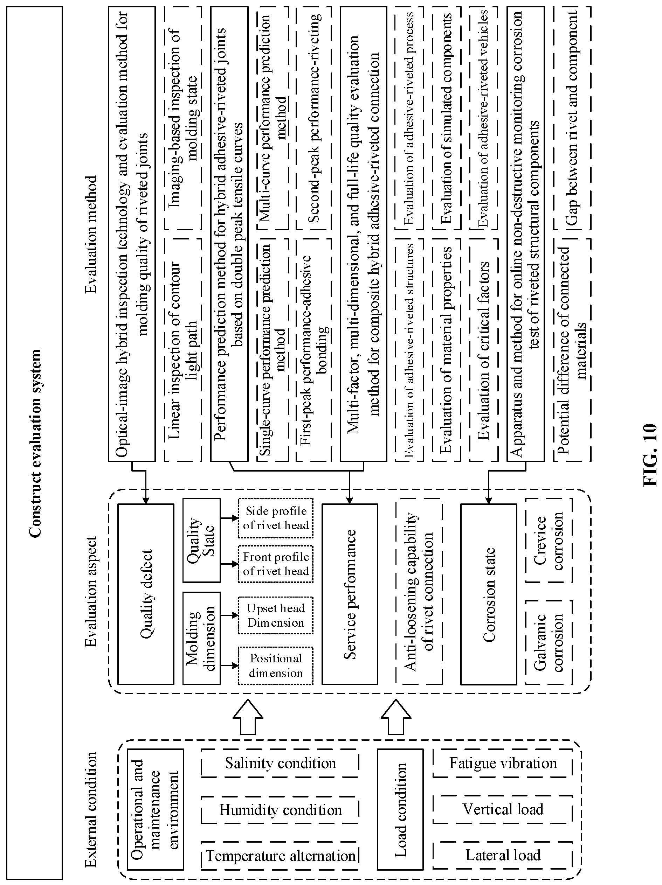

In an embodiment, after the vehicle is assembled, a quality evaluation method for hybrid adhesive-riveted connection is developed based on unique structural characteristics of rail vehicles, the complex operational and maintenance environment (such as alternating temperatures, humidity conditions, atmospheric salinity corrosion conditions), and load conditions (including lateral loads, vertical loads, fatigue vibration loads, and the like.) of trains. The quality evaluation method encompasses evaluation of factors such as quality defects, service performance, and corrosion state, and a quality evaluation system is established, as illustrated in FIG. 10. In an embodiment, quality defect verification, and/or service performance verification, and/or corrosion state verification are performed on the vehicle after the assembling and manufacturing.

The quality defects may include molding dimensions (such as positional dimensions, upset head dimensions) and the quality state of rivets after formation (including the front and side profiles of the rivet heads). The riveted molding quality is detected using a hybrid optical imaging inspection technique for riveted joint molding quality. This technique consists of linear optical detection of riveted joint contours and image-based detection of rivet joint molding state, and includes four procedures: quality coordinate system establishment, data acquisition, quality evaluation, and quality recording. Surface contour dimensions and the quality state are detected, and the quality state at each position is accurately recorded to facilitate quality recording.

The service performance is primarily manifested in the ability of a rivet to resist loosening. The service performance can be evaluated by using performance prediction methods for adhesive-riveted composite joints based on double peak tensile curves, which include a "single-curve joint performance prediction method" and a "multi-curve joint performance prediction method." The methods focus on analyzing the tensile force-displacement curves of a single curve and multiple curves to predict the joint performance at a first peak (adhesive bonding) and a second peak (riveting), thereby reflecting the corresponding service performance. In an embodiment, the single-curve joint performance prediction method involves predicting the performance of the riveted joint in stages based on the adhesive bonding curve and the riveting curve. The multi-curve joint performance prediction method involves analyzing and comparing the dispersion of the curves at various stages based on multiple sets of repetitive joint curves, to predict the stability of the adhesive-riveted joint performance.

In practice, a multi-factor, multi-dimensional, and full-life quality evaluation method for composite adhesive-riveted connection can be established. The method encompasses evaluations of adhesive-riveted performance, material properties, key adhesive-riveted quality factors, riveting process, simulation components, and riveted vehicle. Evaluation results reflect the service performance of adhesive-riveted joints. Quality evaluation is performed on the composite adhesive-riveted joints. To ensure long-term, full-life service safety, targeted evaluations are performed at various stages, including adhesive-riveted connection structure design, process planning, and first-vehicle trial production. The evaluation results from various stages serve as evaluation inputs for each other, fostering a continuous iterative optimization procedure for the structure, process, and manufacture of adhesive-riveted composite connection.

The corrosion states primarily manifest as galvanic corrosion and crevice corrosion. In the present disclosure, an online non-destructive monitoring testing method is established for corrosion in riveted structural components. Based on the potential differences of connected materials and the characteristics of gaps between rivets and components, a method for adhesive-riveted connection in service under corrosive conditions are developed.

It can be seen that in the present disclosure, composite information respectively corresponding to each type of composite components among all types of composite components in a vehicle body is determined, and riveting process design information respectively corresponding to each riveted structure of riveted structures is determined, where the riveting process design information includes at least two of riveting design information, procedure qualification information and procedure specification information. Further, riveting procedure process design information respectively corresponding to each riveted structure of the riveted structures is determined, and a vehicle is assembled from the all types of composite components and the riveted structures based on the composite information, the riveting process design information and the riveting procedure process design information. Vehicle quality verification is further performed on the vehicle after assembly. In response to the vehicle quality verification being passed, the composite information respectively corresponding to each type of composite components among the all types of composite components, and the riveting process design information and the riveting procedure process design information respectively corresponding to each riveted structure of the riveted structures are determined. In this way, process design information at every stage is determined, facilitating the manufacture of qualified vehicles based on the determined information. A complete process design method is provided according to the present disclosure, which is conducive to ensuring the performance and quality of adhesive-riveted connection.

In the present disclosure, the method for using composite materials is developed based on the anisotropy and microstructural differences of the material matrix, and the structural characteristics and service environment of the vehicle. This facilitates accurately determining the composite information respectively corresponding to each type of composite components, thereby improving the quality of adhesive-riveted composite connection. By analyzing the manufacturing procedure of adhesive-riveted composite connection, a method of "simulation + information platform + experimental verification" is used, and a method and a system for designing a digital process are formed, which involve riveting design, process evaluation, specification establishment, skill evaluation, and the like. A series of process methods are established for flexible precision assembly of large composite components, high-precision drilling of composite laminates, control of high-performance adhesive bonding interfaces, and highly reliable riveting processes. Based on core procedures such as assembly, drilling, adhesive application, riveting, and testing of large composite components, specific tooling can be developed featuring precise positioning - flexible clamping - integrated lifting - sequential transfer - intelligent assembly. In addition, a series of riveting manufacturing supporting apparatuses, such as efficient drilling apparatuses featuring rapid positioning - mutual constraint - self-feeding, can be developed. Based on the unique structural forms of rail vehicles and the complex operational and maintenance environment and load conditions of trains, a method for quality evaluation and performance assessment of adhesive-riveted composite connection can be established, encompassing factors such as quality defects, service performance, and corrosion states, and a corresponding evaluation system is constructed.

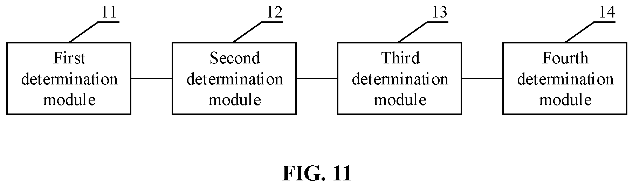

Reference is made to FIG. 11, which is a schematic structural diagram of an apparatus for designing a hybrid adhesive-riveted manufacturing process for composite components according to an embodiment of the present disclosure. The apparatus includes:
a first determination module 11, configured to determine composite information respectively corresponding to each type of composite components among all types of composite components in a vehicle body;
a second determination module 12, configured to determine riveting process design information respectively corresponding to each riveted structure of riveted structures, where the riveting process design information includes at least two of riveting design information, procedure qualification information and procedure specification information;
a third determination module 13, configured to determine riveting procedure process design information respectively corresponding to each riveted structure of the riveted structures; and
a fourth determination module 14, configured to perform vehicle quality verification on a vehicle assembled from the all types of composite components and the riveted structures based on the composite information, the riveting process design information and the riveting procedure process design information, and determine, in response to the vehicle quality verification being passed, the composite information respectively corresponding to each type of composite components among the all types of composite components, and the riveting process design information and the riveting procedure process design information respectively corresponding to each riveted structure of the riveted structures.

In an embodiment, the composite information includes at least two of a composite type, a composite molding manner and a hybrid connection manner,
where the first determination module 11 includes:
a first determination unit, configured to: in response to a main component of the vehicle body being a composite vehicle body for a low-speed urban rail vehicle platform, determine that a composite type of the main component of the vehicle body is a thin-walled integral load-bearing riveted-structure composite,
in response to the main component being an underframe, determine that a composite molding manner of the underframe is pultrusion molding, and determine that a hybrid connection manner of the underframe is adhesive-riveted connection;
in response to the main component being a side wall, determine that a composite molding manner of a upper side sill of the side wall is pultrusion molding, determine that the composite molding manner of a side wall unit of the side wall is lay-up molding, and determine that a hybrid connection manner of the side wall is adhesive-riveted connection; in response to the main component being a roof, determine that a composite molding manner of the roof is lay-up molding, and determine that a hybrid connection manner of the roof is adhesive-riveted connection; and
in response to the main component being an end wall, determine that a composite molding manner of the end wall is integrated lay-up molding, and determine that a hybrid connection manner of the end wall includes adhesive-riveted connection;
a second determination unit, configured to: in response to the main component of the vehicle body being a composite vehicle body for a high-speed train vehicle platform, determine that the composite type of the main component of the vehicle body is the thin-walled integral load-bearing riveted-structure composite,
in response to the main component being the underframe, determine that the composite molding manner of the underframe is pultrusion molding, and determine that the hybrid connection manner of the underframe is adhesive-riveted connection;
in response to the main component being the side wall, determine that the composite molding manner of the side wall is pultrusion molding, and determine that the hybrid connection manner of the side wall is adhesive bonding;
in response to the main component being the roof, determine that the composite molding manner of the roof is pultrusion molding, and determine that the hybrid connection manner of the roof is adhesive bonding; and
in response to the main component being the end wall, determine that the composite molding manner of the end wall is integrated lay-up molding, and determine that the hybrid connection manner of the end wall includes adhesive-riveted connection; and
a third determination unit, configured to: in response to the main component of the vehicle body being a composite-material bogie component of a train, determine that the composite type of the main component of the vehicle body is a thick-walled integral load-bearing riveted-joint composite, determine that the composite molding manner of the main component of the vehicle body is interwoven winding molding, and determine that the hybrid connection manner of the main component of the vehicle body is adhesive-riveted connection.

In an embodiment, the riveting design information includes riveted-structure design information and riveted-joint design information,
where the second determination module 12 includes:
a fourth determination unit, configured to design the riveted structures based on a product design requirement to obtain the riveted-structure design information that satisfies the product design requirement;
a fifth determination unit, configured to design a riveted joint based on the riveted-structure design information to obtain the riveted-joint design information;
a sixth determination unit, configured to determine a key quality characteristic based on the riveted-joint design information, and perform riveting process evaluation on the key quality characteristic to obtain the procedure qualification information; and
a seventh determination unit, configured to determine the procedure specification information based on the riveted-joint design information and the procedure qualification information.

In an embodiment, the riveting procedure process design information includes assembly accuracy information, interface adjustment process, riveting hole preparation process, and blind riveting process.

In an embodiment, the third determination module 13 includes:
an eighth determination unit, configured to determine an assembly deviation threshold corresponding to each type of assembly component, and determine assembly deviation thresholds respectively corresponding to each type of assembly components among all types of assembly components as the assembly accuracy information;
a ninth determination unit, configured to determine that the interface adjustment process for the riveted structure includes performing, based on an assembly requirement of the riveted structure, assembly gap compensation on a connection interface of the riveted structure;
a tenth determination unit, configured to determine that the riveting hole preparation process for the riveted structure includes adding a glass fiber layer on an inner surface of a profile, and performing combined riveting hole preparation in the profile and the glass fiber layer; and
an eleventh determination unit, configured to determine that the blind riveting process for the riveted structure includes determining a riveting sequence of a multi-faceted blind riveted structure, performing, based on a load displacement curve of blind riveting, quality verification on the riveting procedure after performing riveting procedure based on the riveting sequence, and removing a locked spindle blind rivet.

In an embodiment, the apparatus further includes:
an assembly module, configured to assemble and manufacture all types of composite components in the vehicle body through a preset assembly device based on the composite information, the riveting process design information, and the riveting procedure process design information; and
an evaluation module, configured to evaluate the whole vehicle after the assembling and manufacturing.

In an embodiment, the evaluation module is configured to:
perform quality defect verification, and/or service performance verification, and/or corrosion state verification on the vehicle after the assembling and manufacturing.

The apparatus for designing a hybrid adhesive-riveted manufacturing process for composite components according to this embodiment exhibits the same advantages as the method for designing a hybrid adhesive-riveted manufacturing process for composite components according to the above embodiments. In addition, for details about the method for designing a hybrid adhesive-riveted manufacturing process for composite components involved in this embodiment, reference is made to the above embodiments, which is not repeated herein.

A process management system of riveting manufacturing mentioned above is described from a perspective of functional modules. Further, an electronic device is provided according to the present disclosure, which is described from a perspective of hardware. The electronic device according to an embodiment of the present disclosure includes:
a memory, configured to store a computer program; and
a processor, configured to execute the computer program to perform the method for designing a hybrid adhesive-riveted manufacturing process for composite components as described above.

Based on the above embodiments, a computer-readable storage medium is further provided according to an embodiment of the present disclosure. The computer-readable storage medium stores a computer program. The computer program, when executed by a processor, causes the processor to perform the method for designing a hybrid adhesive-riveted manufacturing process for composite components as described above.

The embodiments in this specification are described in a progressive manner, and each embodiment focuses on its differences from other embodiments. The same or similar parts between the embodiments may be referred to each other. Since the apparatus disclosed in the embodiments corresponds to the method disclosed in the embodiments, the description for the apparatus is simple, and reference may be made to the method embodiments for the relevant parts.

It should be further noted that relation terms such as "first" and "second" herein are only used to distinguish one entity or operation from another entity or operation, and do not necessarily require or imply that there is an actual relation or sequence between these entities or operations. Furthermore, the term "include", "include" or any other variation thereof is intended to cover a non-exclusive inclusion, so that a process, a method, an article, or a device including a set of elements includes not only those elements, but also other elements not expressly listed or elements inherent in such a process, method, article, or device. Unless expressly limited, the phrase "including (including) a/an ..." does not exclude existence of other similar elements in the process, method, article or device including the elements.

The steps of the method or algorithm described in conjunction with the embodiments disclosed herein may be directly implemented by hardware, a software module executed by a processor, or a combination thereof. The software module may be arranged in a storage medium known in the art, such as a random access memory (RAM), a memory, a read-only memory (ROM), an electrically programmable ROM, an electrically erasable programmable ROM, a register, a hard disk, a removable disk, and a CD-ROM.

According to the description of the disclosed embodiments, those skilled in the art can implement or use the present disclosure. Various modifications to these embodiments are apparent to those skilled in the art, and the general principles defined herein can be implemented in other embodiments without departing from the spirit and scope of the present disclosure. Therefore, the present disclosure is not be limited to the embodiments described herein, but conforms to the widest scope in accordance with the principle and novel features disclosed herein.

## Claims

1. A method for manufacturing hybrid adhesive-riveted connection for composite components, comprising:
determining a composite parameter respectively corresponding to each type of composite components among all types of composite components in a vehicle body of a to-be-assembled vehicle, wherein the vehicle is assembled from the all types of composite components and riveted structures;
determining riveting process information respectively corresponding to each riveted structure of the riveted structures, wherein the riveting process information comprises at least two of a riveting parameter, a procedure qualification result and a procedure specification;
determining a riveting procedure process parameter respectively corresponding to each riveted structure of the riveted structures;
performing vehicle quality verification on the vehicle based on the composite parameter, the riveting process information and the riveting procedure process parameter; and
manufacturing, in response to the vehicle quality verification being passed, the hybrid adhesive-riveted connection for composite components based on the composite parameter, the riveting process information and the riveting procedure process parameter.

2. The method according to claim 1, wherein the composite parameter comprises at least two of a composite type, a composite molding manner and a hybrid connection manner,
wherein the determining a composite parameter respectively corresponding to each type of composite components among all types of composite components in a vehicle body of a to-be-assembled vehicle comprises:
in response to a main component of the vehicle body being a composite vehicle body for a low-speed urban rail vehicle platform, determining that a composite type of the main component of the vehicle body is a thin-walled integral load-bearing riveted-structure composite,
in response to the main component being an underframe, determining that a composite molding manner of the underframe is pultrusion molding, and determining that a hybrid connection manner of the underframe is adhesive-riveted connection;
in response to the main component being a side wall, determining that a composite molding manner of a upper side sill of the side wall is pultrusion molding, determining that the composite molding manner of a side wall unit of the side wall is lay-up molding, and determining that a hybrid connection manner of the side wall is adhesive-riveted connection;
in response to the main component being a roof, determining that a composite molding manner of the roof is lay-up molding, and determining that a hybrid connection manner of the roof is adhesive-riveted connection;
in response to the main component being an end wall, determining that a composite molding manner of the end wall is integrated lay-up molding, and determining that a hybrid connection manner of the end wall comprises adhesive-riveted connection;
in response to the main component of the vehicle body being a composite vehicle body for a high-speed train vehicle platform, determining that the composite type of the main component of the vehicle body is the thin-walled integral load-bearing riveted-structure composite,
in response to the main component being the underframe, determining that the composite molding manner of the underframe is pultrusion molding, and determining that the hybrid connection manner of the underframe is adhesive-riveted connection;
in response to the main component being the side wall, determining that a composite molding manner of the side wall is pultrusion molding, and determining that the hybrid connection manner of the side wall is adhesive bonding;
in response to the main component being the roof, determining that the composite molding manner of the roof is pultrusion molding, and determining that the hybrid connection manner of the roof is adhesive bonding;
in response to the main component being the end wall, determining that the composite molding manner of the end wall is integrated lay-up molding, and determining that the hybrid connection manner of the end wall comprises adhesive-riveted connection; and
in response to the main component of the vehicle body being a composite-material bogie component of a train, determining that the composite type of the main component of the vehicle body is a thick-walled integral load-bearing riveted-joint composite, determining that the composite molding manner of the main component of the vehicle body is interwoven winding molding, and determining that the hybrid connection manner of the main component of the vehicle body is adhesive-riveted connection.

3. The method according to claim 1 or 2, wherein the riveting parameter comprises riveted-structure information and riveted-joint information, and
the determining riveting process information respectively corresponding to each riveted structure of riveted structures comprises:
obtaining the riveted-structure information based on a product design requirement for the riveted structures;
obtaining the riveted-joint information based on the riveted-structure information;
determining a key quality characteristic based on the riveted-joint information, and performing riveting process evaluation on the key quality characteristic to obtain the procedure qualification result; and
determining the procedure specification based on the riveted-joint information and the procedure qualification result.

4. The method according to any of the preceding claims, wherein the riveting procedure process parameter comprises a assembly accuracy parameter, interface adjustment process, riveting hole preparation process, and blind riveting process.

5. The method according to claim 4, wherein the determining riveting procedure process parameter respectively corresponding to each riveted structure of the riveted structures comprises:
determining an assembly deviation threshold corresponding to each type of assembly component in the vehicle body, and determining assembly deviation thresholds respectively corresponding to each type of assembly components among all types of assembly components as the assembly accuracy parameter;
determining that the interface adjustment process for the riveted structure comprises performing, based on an assembly requirement of the riveted structure, assembly gap compensation on a connection interface of the riveted structure;
determining that the riveting hole preparation process for the riveted structure comprises adding a glass fiber layer on an inner surface of a profile, and performing combined riveting hole preparation in the profile and the glass fiber layer; and
determining that the blind riveting process for the riveted structure comprises determining a riveting sequence of a multi-faceted blind riveted structure, performing, based on a load displacement curve of blind riveting, quality verification on the riveting procedure after performing riveting procedure based on the riveting sequence, and removing a locked spindle blind rivet.

6. The method according to claim 5, further comprising:
assembling all types of composite components in the vehicle body through a preset assembly device based on the composite parameter, the riveting process information, and the riveting procedure process parameter to manufacture the vehicle; and
evaluating the whole vehicle after the assembling.

7. The method according to claim 6, wherein the evaluating the whole vehicle after the assembling comprises:
verifying quality defect of the vehicle after the assembling; and/or
verifying service performance of the vehicle after the assembling; and/or
verifying corrosion state of the vehicle after the assembling.

8. An apparatus for manufacturing hybrid adhesive-riveted connection for composite components, comprising:
a first determination module, configured to determine a composite parameter respectively corresponding to each type of composite components among all types of composite components in a vehicle body of a to-be-assembled vehicle, wherein the vehicle is assembled from the all types of composite components and riveted structures;
a second determination module, configured to determine riveting process information respectively corresponding to each riveted structure of the riveted structures, wherein the riveting process information comprises at least two of a riveting parameter, a procedure qualification result and a procedure specification;
a third determination module, configured to determine a riveting procedure process parameter respectively corresponding to each riveted structure of the riveted structures; and
a fourth determination module, configured to perform vehicle quality verification on the vehicle based on the composite parameter, the riveting process information and the riveting procedure process parameter, and manufacture, in response to the vehicle quality verification being passed, the hybrid adhesive-riveted connection for composite components based on the composite parameter, the riveting process information and the riveting procedure process parameter.

9. An electronic device, comprising:
a memory, configured to store a computer program; and
a processor, configured to execute the computer program to perform the method according to any one of claims 1 to 7.

10. A computer-readable storage medium, storing a computer program, wherein the computer program, when executed by a processor, causes the processor to perform the method according to any one of claims 1 to 7.
